# EUROPÄISCHE PATENTANMELDUNG

(11) **EP 2 685 397 A1**
(43) Veröffentlichungstag der Anmeldung: **15.01.2014**
(21) Anmeldenummer: 13002860.8
(22) Anmeldetag: 04.06.2013
(51) Int. Cl.: G06F 19/00

(54) **System zur schnellen und optimierten Bearbeitung medizinischer Proben**

(30) Priorität: 10.07.2012 DE 202012006683 U; 07.12.2012 DE 202012009394 U
(71) Anmelder: Labor Krone GbR, 32105 Bad Salzuflen (DE)
(72) Erfinder: Dieter, Münstermann Dr. med., 32049 Herford (DE)
(74) Vertreter: Kayser, Christoph

(57) **Zusammenfassung**

Ein Barcodesystem zur Proben- und Auftragskennzeichnung durch einen Auftraggeber, mit einer Einrichtung zum Erzeugen von Barcodes in einem Labor, mit einer Einrichtung zum Bereitstellen von Etiketten in dem Labor, mit einer Einrichtung zum Drucken der erzeugten Barcodes auf die bereitgestellten Etiketten und mit einer Bindeeinrichtung zur Erzeugung eines Barcode-Heftes mit einer Mehrzahl unterschiedlicher Barcodes für den Auftraggeber.

## Beschreibung

Die vorliegende Erfindung betrifft ein Barcodesystem zur Proben- und Auftragskennzeichnung durch einen Auftraggeber gemäß Anspruch 1.

Aus dem Stand der Technik sind Barcodesysteme bekannt, um Proben und Aufträge zu kennzeichnen. In der Regel werden eingehende Proben und Aufträge mit einem Barcodeetikett versehen, so dass die Proben und Aufträge während einer weiteren Bearbeitung jederzeit identifizierbar sind und gegebenenfalls über die Identifikation einer auftragsgemäßen Bearbeitung zugeführt werden können.

Solche Systeme regeln die Bearbeitung von Proben und Aufträgen bei einem Auftragnehmer.

Es hat sich aber gezeigt, dass die Bearbeitung insbesondere medizinischer Proben abhängig ist von Angaben des Auftraggebers, die bei bisher üblichen Systemen in einen Barcode übertragen werden müssen. Dies ist aufwändig und nicht fehlerfrei durchzuführen.

Die Aufgabe der vorliegenden Erfindung ist daher, ein Barcodesystem zu entwickeln, bei dem die Identifikation von Proben und Aufträgen bereits auftraggeberseitig durchgeführt wird.

Die Aufgabe wird erfindungsgemäß dadurch gelöst, dass das Barcodesystem eine Einrichtung zum Erzeugen von Barcodes in einem Labor, eine Einrichtung zum Bereitstellen von Etiketten in dem Labor, eine Einrichtung zum Drucken der erzeugten Barcodes auf die bereitgestellten Etiketten und eine Bindeeinrichtung zur Erzeugung eines Barcodeheftes für den Auftraggeber umfasst.

Mit der vorliegenden Erfindung ist es möglich, dem Auftraggeber ein Barcodeheft mit laborseitig hergestellten Barcodeetiketten zur Verfügung zu stellen, so dass der Auftraggeber bereits Proben und Aufträge mit vorbestimmten Etiketten versehen kann. Bei Anlieferung der Proben und Aufträge im Labor kann dann die auftragsgemäße Bearbeitung sofort beginnen.

Ein weiterer Vorteil der vorliegenden Erfindung ist, dass ein Markierungsformular und wenigstens eine Probe vorgesehen sind, wobei auf dem Markierungsformular ein mit einem ersten der Barcodes versehenes erstes Etikett aufgebracht ist und auf der Probe ein mit dem ersten der Barcodes versehenes zweites Etikett aufgebracht ist, derart, dass das Markierungsformular der Probe zugeordnet werden kann. Durch das Markierungsformular ist es möglich, eine visuelle Überprüfung der durch den Auftraggeber verwendeten Barcodes vorzunehmen. Eine Übereinstimmung der Barcodes auf der Probe und dem Markierungsformular gewährleistet die auftragsgemäße Bearbeitung der Probe.

Ein weiterer Vorteil der vorliegenden Erfindung ist, dass eine Steuereinrichtung die Einrichtung zum Bereitstellen von Etiketten derart steuert, dass eine erste Mehrzahl größerer Etiketten und eine zweite Mehrzahl kleinerer Etiketten bereitgestellt werden. Dadurch ist es möglich, die Etiketten auf unterschiedlichen vorgegebenen Feldern anzubringen und dem Auftraggeber die Anbringung der richtigen Etiketten zu erleichtern.

Ein weiterer Vorteil der vorliegenden Erfindung ist, dass die Steuereinrichtung die Einrichtung zum Erzeugen von Barcodes zur Erzeugung einer der ersten Mehrzahl größerer Etiketten und der zweiten Mehrzahl kleinerer Etiketten entsprechenden Anzahl erster Barcodes steuert. Dadurch wird gewährleistet, dass immer eine entsprechende Anzahl gleicher Barcodes für eine erste Mehrzahl größerer Etiketten und eine zweite Mehrzahl kleinerer Etiketten vorhanden ist.

Ein weiterer Vorteil der vorliegenden Erfindung ist, dass die Steuereinrichtung die Einrichtung zum Drucken der erzeugten Barcodes so steuert, dass die erste Mehrzahl größerer Etiketten und die zweite Mehrzahl kleinerer Etiketten gedruckt werden. Dadurch wird gewährleistet, dass nicht nur die entsprechende Anzahl von Barcodes erzeugt, sondern auch auf die Etiketten der jeweiligen ersten Mehrzahl oder zweiten Mehrzahl aufgebracht werden.

Ein weiterer Vorteil der vorliegenden Erfindung ist, dass die erste Mehrzahl größerer Etiketten Acht beträgt. Es hat sich gezeigt, dass für eine optimierte Bearbeitung von Proben und Aufträgen acht größere Etiketten mit den entsprechenden Barcodes vorteilhaft sind.

Ein weiterer Vorteil der vorliegenden Erfindung ist, dass die zweite Mehrzahl kleinerer Etiketten Fünf beträgt. Auch hier hat sich gezeigt, dass für die optimierte Bearbeitung von Proben und Aufträgen die Anzahl Fünf eine geeignete Menge für die zweite Mehrzahl kleinerer Etiketten ist.

Ein weiterer Vorteil der vorliegenden Erfindung ist, dass die Einrichtung zur Erzeugung von Barcodes diese mit einer zwölfstelligen Nummer erzeugen. Dadurch wird eine fehlerfreie Identifikation von Probenaufträgen gewährleistet.

Ein weiterer Vorteil der vorliegenden Erfindung ist, dass der Barcode eine Standortkennung, eine Kundennummer/Arztkennung, eine Proben-Nummer 1 bis 9999 sowie eine Objektkennung für 99 Dokumente oder Materialien umfasst. Auch damit ist eine optimierte Bearbeitung gewährleistet.

Ein weiterer Vorteil der vorliegenden Erfindung ist, dass eine Einrichtung zum Scannen von Barcodes die auf dem Markierungsformular und der Probe aufgebrachten Barcodes scannt und eine Datenerfassungseinrichtung die aus den gescannten Barcodes umgewandelten Daten speichert. Durch die Speicherung der Daten können diese zum Beispiel zeitversetzt von der Steuereinrichtung genutzt werden.

Ein weiterer Vorteil der vorliegenden Erfindung ist, dass die Datenerfassungseinrichtung mit einer Verteileinrichtung verbunden ist und die Steuereinrichtung die Proben über die Verteileinrichtung wenigstens zwei Bearbeitungsstationen zuweisen. Durch diese Maßnahme wird eine automatische Bearbeitung ermöglicht. In Abhängigkeit der Identifikation der Proben auf Aufträge können die Proben über die Verteileinrichtung den jeweiligen Bearbeitungsstationen zugeführt werden.

Dazu ist es von Vorteil, dass die Verteileinrichtung eine maschinelle Verbindung zwischen der Einrichtung zum Scannen und der wenigstens zwei Bearbeitungsstationen herstellt. So können die Proben nach dem Scannen und Speichern der Daten maschinell der jeweiligen Bearbeitungsstation zugeführt werden.

Schließlich ist es auch von Vorteil, dass die Steuereinrichtung in Abhängigkeit von dem jeweils eingescannten Barcode einen Gerätepark zur automatischen Bearbeitung der betreffenden Probe steuert. Dies ist insbesondere dann von Vorteil, wenn an bestimmten Bearbeitungsstationen unterschiedliche Bearbeitungsvorgänge durchgeführt werden können.

Ein besonderer Vorteil ist die Art der Probenverteilung und die optimierte Probenbearbeitung, die durch eine Kombination von paralleler und sequenzieller Bearbeitung erreicht wird.

Ein weiterer Vorteil der vorliegenden Erfindung ist die Skalierbarkeit des Verfahrens, die eine schnelle Anpassung der Untersuchungskapazitäten an Auftragszahlen ermöglicht.

Eine Ausführungsform der vorliegenden Erfindung wird im Folgenden beschrieben.

Das Barcodesystem zur Proben- und Auftragskennzeichnung durch einen Auftraggeber umfasst eine Einrichtung zum Erzeugen von Barcodes in einem Labor, eine Einrichtung zum Bereitstellen von Etiketten in dem Labor, eine Einrichtung zum Drucken der erzeugten Barcodes auf die bereitgestellten Etiketten und eine Bindeeinrichtung zum Erzeugen eines Barcodeheftes mit einer Mehrzahl unterschiedlicher Barcodes für den Auftraggeber. Die vorgenannten Einrichtungen werden gesteuert von einer Steuereinrichtung, zum Beispiel einem Rechner. Die Einrichtungen sind über übliche Verbindungselemente, zum Beispiel Kabel, mit dem Rechner verbunden oder in anderen Ausführungsformen auch in den Rechner integriert. Die Steuerung erfolgt mit Hilfe von Software.

Das laborseitig hergestellte Barcodeheft enthält Anweisungen an den Auftraggeber, die darin enthaltenen Barcodeetiketten richtig zu verwenden. So befinden sich in der vorliegenden Ausführungsform alle Barcodes für einen Auftrag/Patient in einer Reihe. Überzählige Barcodes einer Reihe dürfen nicht für weitere Aufträge/Patienten verwendet werden.

Es ist günstig, wenn der Auftraggeber das Barcodeheft als Laborjournal verwendet, in dem Patientendaten auf der linken Seite manuelle eingetragen werden. An dem Bereich für die manuelle Eintragung der Patientendaten befindet sich ein Barcode, der direkt in eine Praxis-EDV eingescannt werden kann, wenn als Befundweg die Datenfernübertragung genutzt werden soll.

In einer vorteilhaften Ausführungsform stellt jede Reihe von Barcodes eine erste Gruppe von Barcodes bzw. erste Barcodes dar. Jede weitere Reihe bildet eine numerisch höher gestufte Gruppe von Barcodes. So gibt es also in einem Barcodeheft erste Barcodes, zweite Barcodes, dritte Barcodes in fortlaufender Folge. Die Gruppe der ersten Barcodes oder erste Gruppe von Barcodes sind auf einer ersten Mehrzahl von größeren Etiketten und auf einer zweiten Mehrzahl kleinerer Etiketten gedruckt. In der vorliegenden Ausführungsform umfasst die erste Mehrzahl größerer Etiketten acht Etiketten und die zweite Mehrzahl kleinerer Etiketten umfasst fünf Etiketten. Diese insgesamt dreizehn ersten Barcodes werden erfindungsgemäß als erste Barcodes bezeichnet. Auch diese ersten Barcodes sind unterschiedlich zueinander. Es sind im Sinn der vorliegenden Erfindung erste Barcodes in Bezug auf einen Auftrag/Patienten.

Auch die zweiten, dritten und weiteren Barcodes umfassen in der vorliegenden Ausführungsform insgesamt dreizehn Etiketten, mit jeweils acht größeren Etiketten und fünf kleineren Etiketten. In anderen Ausführungsformen der Erfindung können auch mehr oder weniger als acht größere und fünf kleinere Etiketten verwendet werden. In der vorliegenden Ausführungsform sind die acht größeren und die fünf kleineren Etiketten jeweils untereinander gleich groß. In anderen Ausführungsformen können diese jeweils untereinander auch unterschiedlich groß sein.

In der vorliegenden Ausführungsform wird also für einen Auftrag jeweils eine Gruppe von dreizehn Etiketten (acht große und fünf kleine) verwendet. Zum Beispiel wird für den ersten Auftrag die erste Gruppe Etiketten und für den zweiten Auftrag die zweite Gruppe von Etiketten usw. verwendet.

Die erste Mehrzahl größerer Etiketten ist dafür vorgesehen, in beliebiger Reihenfolge auf den Auftrag und der bevorzugten Ausführungsform insbesondere auf das Markierungsformular (zum Beispiel den Überweisungsschein), auf die Proben (zum Beispiel Röhrchen) und gegebenenfalls auf weitere Dokumente geklebt zu werden. Mit der vorliegenden bevorzugten Ausführungsform können insgesamt acht Objekte beklebt werden. Dadurch besteht eine eindeutige Zuordnung von den mit den ersten Barcodes bzw. der ersten Mehrzahl von Etiketten beklebten Dokumenten zu der jeweiligen Probe des ersten Auftrags.

Bei allen weiteren Aufträgen wird ebenso verfahren.

Die zweite Mehrzahl kleinerer Etiketten liegt in einer Anzahl von Fünf vor. Diese kleineren Etiketten gehören zu dem ersten Auftrag und tragen jeweils unterschiedliche erste Barcodes. Diese unterschiedlichen ersten Barcodes identifizieren Funktionsteste. In der vorliegenden Ausführungsform werden also die Funktionsteste 1 bis 5 identifiziert.

Die Barcodes tragen zudem eine zwölfstellige Nummer. Diese Nummer setzt sich wie folgt zusammen:
<SS><XXXX><YYYY><ZZ>

| | | |
|---|---|---|
| mit | SS | = Standortkennung |
| | XXXX | = Kundennummer/Arztkennung |
| | YYYY | = Proben-Nummer 1 bis 9999 |
| | ZZ | = Objektkennung für 99 Dokumente oder Materialen. |

Das erfindungsgemäße Barcodesystem umfasst auch einen laborseitigen Scanner, der die vom Auftraggeber überreichten Dokumente elektronisch erfasst. Dazu werden spezielle Markierungsformulare verwendet. Durch das erfindungsgemäße Barcodesystem und die Markierungsformulare ist es möglich, das jeweilige Auftragsformular und die jeweilige Probe zu trennen, so dass die Probe sofort nach Eingang verarbeitet werden kann. Die weitere Bearbeitung erfolgt papierlos.

Beim Auftragseingang werden alle Elemente eines Auftrags (Dokumente und Proben) durch eine Scann-Aktion registriert bzw. in einer Datenerfassungseinrichtung gespeichert. Der Scann-Vorgang kann manuell oder automatisch erfolgen. Bei einem automatischen Einscannen erfolgt die Zuordnung für das Gerät durch die Erkennung des Probengefäßes.

Das Barcodesystem lässt eine variable Zuordnung zwischen der durch den Scann-Vorgang ermittelten Probennummer der Registrierung in einer Datenbank der Datenerfassungseinrichtung zu. Die nicht eindeutigen Probennummern erhalten in Kombination mit zum Beispiel dem Auftragsdatum die benötigte Eindeutigkeit. '

Ein weiterer Aspekt des erfindungsgemäßen Barcodesystems ist, dass dieses auch eine Verteileinrichtung umfasst. Diese Verteileinrichtung ist an die Datenerfassungseinrichtung angeschlossen und wird von der Steuereinrichtung gesteuert. Dadurch können die Proben über die Verteileinrichtung wenigstens zwei unterschiedlichen Bearbeitungsstationen zugewiesen werden, an denen die Proben auftragsgemäß bearbeitet werden. Relativ oder zusätzlich können die Proben über die Verteileinrichtung auch einer Bearbeitungsstation zugewiesen werden, an der auftragsgemäß unterschiedliche Bearbeitungsvorgänge durchgeführt werden.

Die Verteileinrichtung umfasst ein maschinelles Verteilsystem, das die Zuführung der jeweiligen Probe zu der betreffenden Bearbeitungsstation sicherstellt. Durch die Möglichkeit, mit dem erfindungsgemäßen Barcodesystem einen gemeinsamen Gerätepark anzusteuern, können die Geräte im Labor optimal ausgenutzt werden.

Die jeweiligen Steuerungen der Steuereinrichtung werden mit Software unterstützt. Ebenso ist über eine sogenannte Middleware eine gemeinsame und gleichzeitige Nutzung der Proben durch verschiedene Auftragnehmer möglich.

Ein weiterer wichtiger Bestandteil der Erfindung ist die Art der Aliquotierung. In medizinischen Laboratorien werden üblicherweise zwei Arbeitsweisen verwendet:

### 1. Die serielle Abarbeitung ohne Aliquotierung

Hier durchläuft eine Probe alle Arbeitsplatz nacheinander. Diese Form der Probenbearbeitung ist zeitaufwändig.

### 2. Die parallele Abarbeitung mit je einem Aliquot für einen Arbeitsplatz

Hier wird für jeden Arbeitsplatz ein Teil der Originalprobe abgefüllt und dem Arbeitsplatz zur Verfügung gestellt. Diese Form der Bearbeitung ist kostenintensiv und hat den Nachteil, dass mehr Probenmaterial erforderlich ist als bei der unter 1. Beschriebenen Arbeitsweise.

Die Erfindung besteht hier in einer Optimierung der Bearbeitungszeit, der Kosten und der optimalen Ausnutzung der verfügbaren Probenmenge durch die Abfüllung eines Aliquotes und der parallelen Bearbeitung beider Probenteile in verschiedenen Laborbereichen, wobei die Bearbeitung in jedem Laborteil wiederum sequenziell ist. Nach der Bearbeitung werden beide Proben schließlich getrennt.

Mit dieser speziellen, aus der Erfindung hervorgegangenen Arbeitsweise ist es gelungen, die Bearbeitungszeit und die Kosten zu optimieren. Außerdem wird durch dieses spezielle Verfahren eine Skalierbarkeit des Bearbeitungsprozesses erreicht, die es ermöglicht, durch Anschaffung zusätzlicher Sortierautomaten und Analysengeräte die Bearbeitungskapazitäten beliebig und den Anforderungen entsprechen heraufzusetzen, ohne eine Einschränkung durch die Kapazitätsgrenzen von kommerziell verfügbaren "Geräte-Straßen-Systemen" zu erfahren.

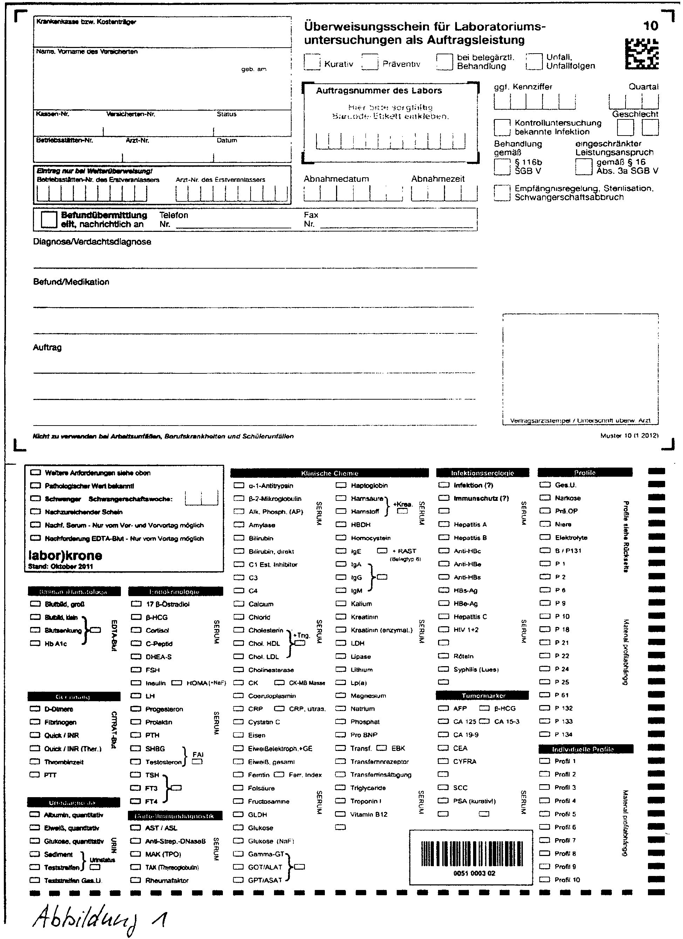

In Abbildung 1 ist ein Muster eines Markierungsformulars als Überweisungsschein dargestellt.

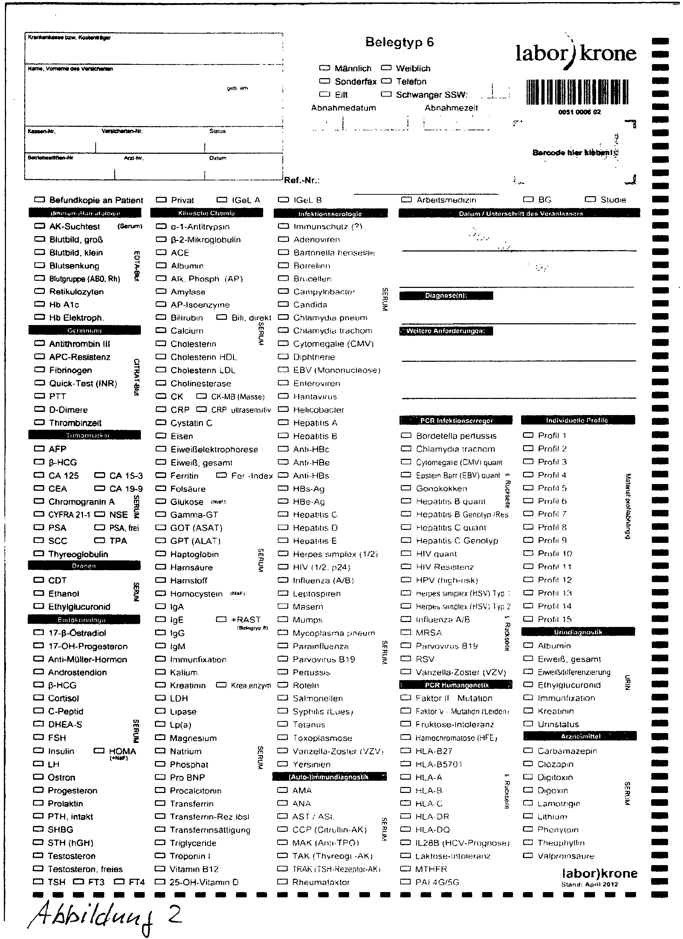

In Abbildung 2 ist ein Beispiel eines Markierungsformulars als Belegtyp 6 dargestellt.

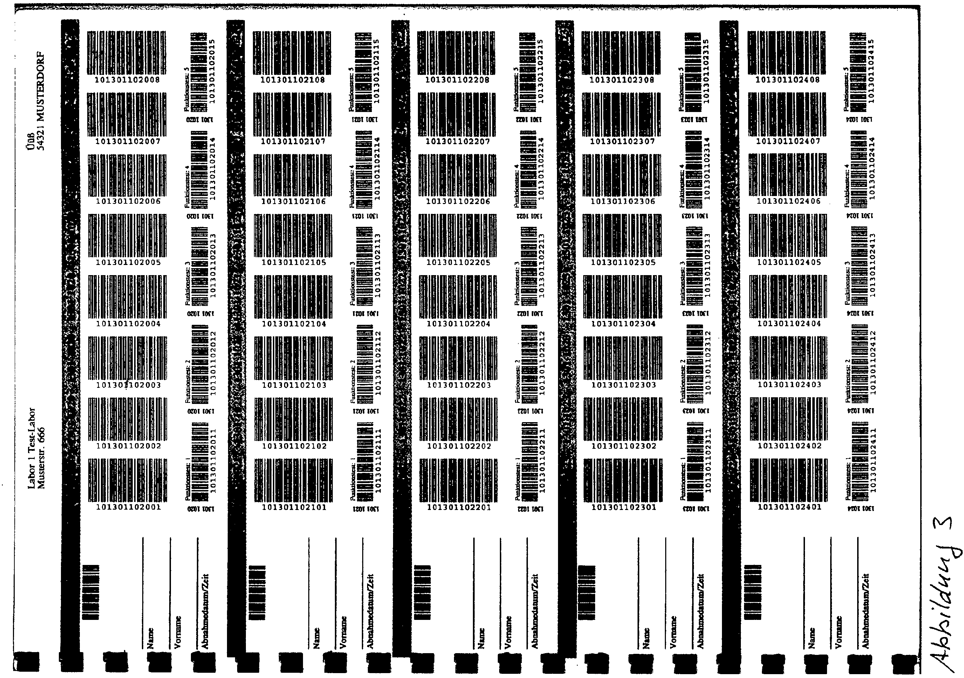

In Abbildung 3 ist die Seite 1 des Barcodeheftes dargestellt.

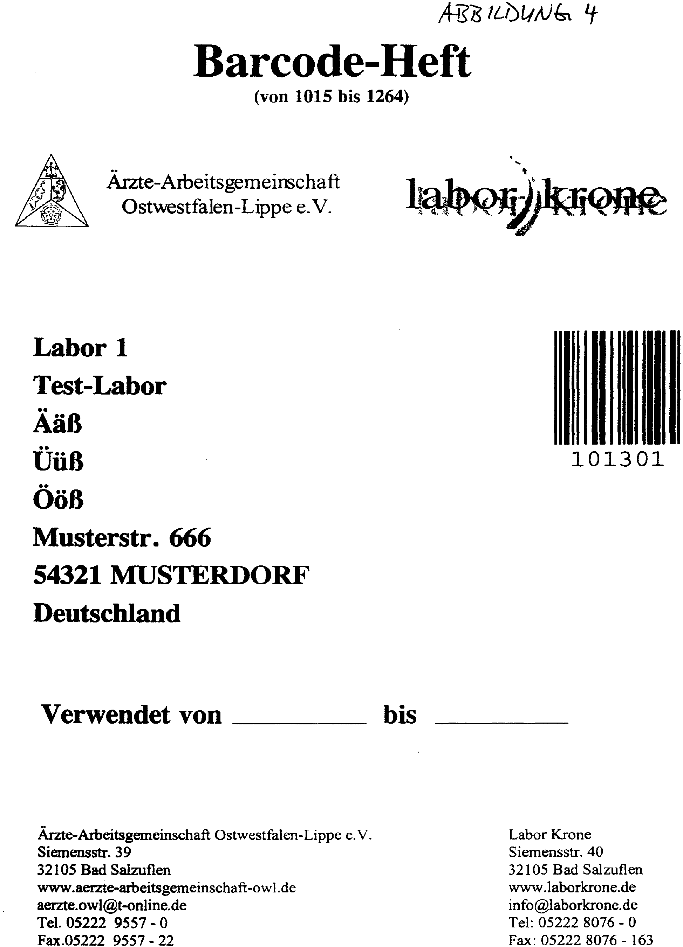

In Abbildung 4 ist die Seite 2 des Barcodeheftes dargestellt.

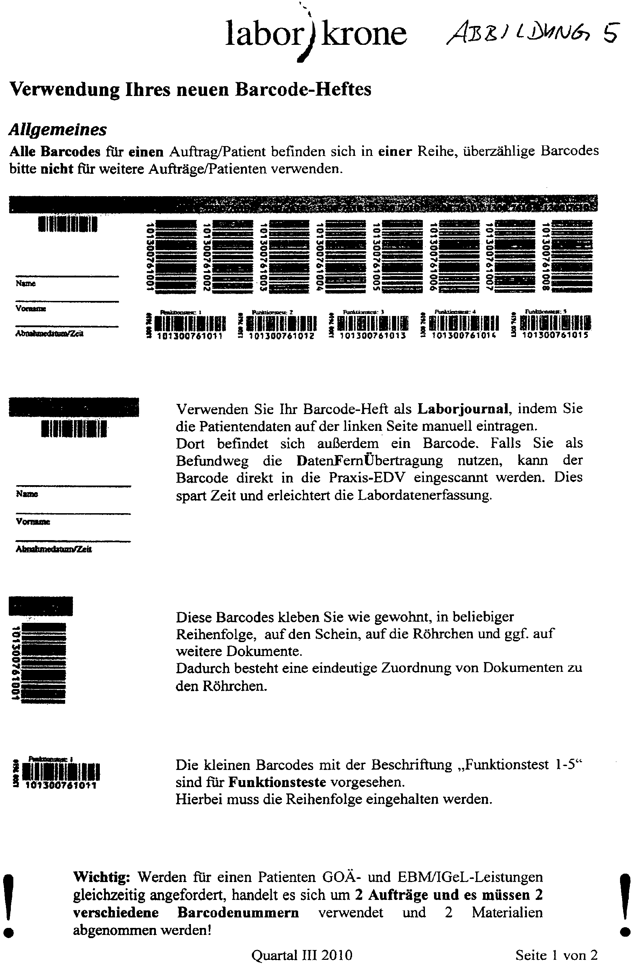

In Abbildung 5 ist eine erste Seite mit fünf Gruppen von jeweils einer ersten Mehrzahl und einer zweiten Mehrzahl von Etiketten dargestellt.

## Patentansprüche

1. Barcodesystem zur Proben- und Auftragskennzeichnung durch einen Auftraggeber, mit einer Einrichtung zum Erzeugen von Barcodes in einem Labor,
mit einer Einrichtung zum Bereitstellen von Etiketten in dem Labor,
mit einer Einrichtung zum Drucken der erzeugten Barcodes auf die bereitgestellten Etiketten und mit einer Bindeeinrichtung zur Erzeugung eines Barcode-Heftes mit einer Mehrzahl unterschiedlicher Barcodes für den Auftraggeber.

2. Barcodesystem nach Anspruch 1,
**gekennzeichnet durch,**
ein Markierungsformular und wenigstens eine Probe, wobei auf dem Markierungsformular ein mit einem ersten der Barcodes versehenes erstes Etikett aufgebracht ist und auf der Probe ein mit dem ersten der Barcodes versehenes zweites Etikett aufgebracht ist, derart, dass das Markierungsformular der Probe zugeordnet werden kann.

3. Barcodesystem nach Anspruch 1 oder 2,
**dadurch gekennzeichnet,**
**dass** eine Steuereinrichtung die Einrichtung zum Bereitstellen von Etiketten derart steuert, dass eine erste Mehrzahl größerer Etiketten und eine zweite Mehrzahl kleinerer Etiketten bereitgestellt werden.

4. Barcodesystem nach Anspruch 3,
**dadurch gekennzeichnet,**
**dass** die Steuereinrichtung die Einrichtung zum Erzeugen von Barcodes zur Erzeugung einer der ersten Mehrzahl größerer Etiketten und der zweiten Mehrzahl kleinerer Etiketten entsprechenden Anzahl erster Barcodes steuert.

5. Barcodesystem nach Anspruch 3,
**dadurch gekennzeichnet,**
**dass** die Steuereinrichtung die Einrichtung zum Drucken der erzeugten Barcodes so steuert, dass die erste Mehrzahl größerer Etiketten und die zweite Mehrzahl kleinerer Etiketten bedruckt werden.

6. Barcodesystem nach einem der Ansprüche 3 bis 5,
**dadurch gekennzeichnet,**
**dass** die erste Mehrzahl größerer Etiketten 8 beträgt.

7. Barcodesystem nach einem der Ansprüche 3 bis 6, **dadurch gekennzeichnet,**
**dass** die zweite Mehrzahl kleinerer Etiketten 5 beträgt.

8. Barcodesystem nach einem der Ansprüche 1 bis 7, **dadurch gekennzeichnet,**
**dass** die Einrichtung zur Erzeugung von Barcodes diese mit einer zwölfstelligen Nummer erzeugen.

9. Barcodesystem nach Anspruch 8,
**dadurch gekennzeichnet,**
**dass** jeder Barcode wie folgt aufgebaut ist:
<SS><XXXX><YYYY><ZZ>
| | | |
|---|---|---|
| mit | SS | = Standortkennung |
| | XXXX | = Kundennummer/Arztkennung |
| | YYYY | = Proben-Nummer 1 bis 9999 |
| | ZZ | = Objektkennung für 99 Dokumente oder Materialen |

10. Barcodesystem nach einem der Ansprüche 2 bis 9,
**dadurch gekennzeichnet,**
**dass** eine Einrichtung zum Scannen von Barcodes die auf dem Markierungsformular und der Probe aufgebrachten Barcodes scannt und eine Datenerfassungseinrichtung die aus den gescannten Barcodes umgewandelten Daten speichert.

11. Barcodesystem nach Anspruch 10,
**dadurch gekennzeichnet,**
**dass** die Datenerfassungseinrichtung mit einer Verteileinrichtung verbunden ist und die Steuereinrichtung die Proben über die Verteileinrichtung wenigstens zwei Bearbeitungsstationen zuweisen.

12. Barcodesystem nach Anspruch 11,
**dadurch gekennzeichnet,**
**dass** die Verteileinrichtung eine maschinelle Verbindung zwischen der Einrichtung zum Scannen und der wenigstens zwei Bearbeitungsstationen herstellt.

13. Barcodesystem nach einem der Ansprüche 10 bis 12,
**dadurch gekennzeichnet,**
**dass** die Steuereinrichtung in Abhängigkeit von dem jeweils eingescannten Barcode einen Gerätepark zur automatischen Bearbeitung der betreffenden Probe steuert.
